# EUROPEAN PATENT SPECIFICATION

(11) **EP 2 903 588 B1**
(45) Date of publication and mention of the grant of the patent: **19.02.2020**
(21) Application number: 13777202.6
(22) Date of filing: 01.10.2013
(51) Int. Cl.: A61K 8/67, A61K 8/73, A61Q 19/08

(54) **DERMAL FILLER HYDROGELS WITH VITAMIN A/CYCLODEXTRIN INCLUSION COMPLEXES**
DERMALE FÜLLSTOFFHYDROGELE MIT VITAMIN-A-/CYCLODEXTRIN-EINSCHLUSSKOMPLEXEN
HYDROGELS DE COMBLEMENT DERMIQUE COMPORTANT DES COMPLEXES D'INCLUSION DE VITAMINE A/CYCLODEXTRINE

(30) Priority: 02.10.2012 US 201261709065 P
(43) Date of publication of application: 12.08.2015
(60) Divisional application: 19219360.5
(73) Proprietor: ALLERGAN, INC., Irvine, CA 92612 (US)
(72) Inventor: LIU, Futian, Sunnyvale, California 94087 (US); YU, Xiaojie, Irvine, California 92602 (US); MANESIS, Nicholas J., Summerland, California 93067 (US)
(74) Representative: Hoffmann Eitle
(86) International application number: PCT/US2013/062886
(87) International publication number: WO 2014/055532

(56) References cited:
- WO-A1-94/21225
- US-A- 5 985 296
- US-A1- 2007 172 442
- US-A1- 2009 018 102
- US-A1- 2011 077 737
- US-A1- 2011 118 206
- ZAWKO SCOTT A ET AL: "Drug-binding hydrogels of hyaluronic acid functionalized with beta-cyclodextrin", JOURNAL OF BIOMEDICAL MATERIALS RESEARCH. PART A, JOHN WILEY & SONS, INC, US, vol. 87, no. 4, 15 December 2008 (2008-12-15), pages 1044-1052, XP002728107, ISSN: 1552-4965
- Nilüfer Tarimci: "7 CYCLODEXTRINS IN THE COSMETIC FIELD" In: "Cyclodextrins in Pharmaceutics, Cosmetics, and Biomedicine: Current and Future Industrial Applications", 28 March 2011 (2011-03-28), John Wiley & Sons, Inc, XP055134329, ISBN: 978-0-47-092681-9 pages 131-144, DOI: DOI: 10.1002/9780470926819.ch7, 4.Applicatio of CDs in Cosmetic Formulations; page 134 - page 137; figure 3; table 4

## Description

### BACKGROUND

Skin aging is a challenge of modern human life. Skin aging may be caused by endogenous (intrinsic and internal) and/or exogenous (environmental) factors. Endogenous factors include genetic mutation, cell metabolism, hormones, etc. Exogenous factors include oxygen, UV, ionizing radiation, chemicals, etc. As skin ages, the epidermis and the terminus become thinner and dermal connective tissue changes. Dermal connective tissue includes an extracellular matrix (principally collagen fibers) and fibroblasts. The fibroblasts lie isolated from one another in the extracellular matrix. As skin ages, the structure of the extracellular matrix is damaged by collagen breakdown or partial fragmentation of the collagen. As a result collagen fragments may not be able to attach to fibroblasts, leading to reduced mechanical tension and shrinkage of the fibroblasts. The reduction of tension may upregulate the formation of matrix metalloproteins and downregulate collagen synthesis. Skin aging may be prevented by regulating the activity of fibroblasts and balancing the formation of collagen synthesis and the matrix metalloproteins. US 2009/0018102 discloses injectable compositions containing hyaluronic acid and 0.00001 % by weight of retinol.

### SUMMARY

Compositions comprising a hyaluronic acid are disclosed herein. These compositions further comprise a cyclodextrin and vitamin A complexed to cyclodextrin. A hyaluronic acid and a cyclodextrin may be separate compounds in a composition, or they may be conjugated to form a single compound, or a conjugate of a hyaluronic acid and a cyclodextrin.

Some embodiments comprise a composition comprising: a hyaluronic acid and a cyclodextrin, or a conjugate of a hyaluronic acid and a cyclodextrin; and a vitamin A; wherein the vitamin A is complexed to the cyclodextrin.

Some embodiments comprise a compound comprising a hyaluronic acid conjugated to a cyclodextrin.

Some embodiments comprise a dermal aesthetic product comprising a composition described herein. The present invention is defined by the appended claims.

### DETAILED DESCRIPTION

Vitamin A includes retinoic acid, including trans-retinoic acid, and compounds that may be converted to retinoic acid *in vivo,* such as retinoic acid esters, retinol, and retinyl esters. Vitamin A may be used as a cosmetic ingredient to stimulate skin fibroblasts to produce procollagen I and III and at the same time to downregulate the formation of the collagen-degrading enzymes (matrix-metalloproteinases). Retinoic acid can only be used in prescription medicine because of its side effects. However, retinol and retinyl esters can be metabolized by skin into retinoic acid and have been approved for cosmetic applications. Retinyl esters may be more stable than retinol and may be more widely used as cosmetic ingredients. Though retinyl esters may be used as cosmetic ingredients in creams or lotions, ester forms of vitamin A, including retinyl palmitate, retinyl acetate and retinyl propionate , are used less extensively in hyaluronic acid compositions such as dermal fillers. This may be because vitamin A esters are insoluble in aqueous environments, and because sustained release of vitamin A esters may be difficult to achieve.

Complexing a vitamin A with a cyclodextrin may help to improve the solubility of the vitamin A and/or improve sustained release of vitamin A. Such a complex may comprise a vitamin A complexed to a cyclodextrin where hyaluronic acid and cyclodextrin are not conjugated, or hyaluronic acid and cyclodextrin may be separate molecules; or vitamin A may be complexed to a cyclodextrin that is conjugated to a hyaluronic acid, or hyaluronic acid and cyclodextrin may be joined by one or more linking bonds or groups.

Any vitamin A may be complexed, including retinoic acid and its esters, such as methyl retinoate, ethyl retinoate, propyl retinoate, isopropyl retinoate, butyl retinoate or an isomer thereof, etc.; retinol and its esters (retinyl esters), such as retinyl esters of C₁₋₃₀ carboxylic acids, including lower carboxylic acid esters such as retinyl acetate, retinyl propionate or an isomer thereof, retinyl butyrate or an isomer thereof, etc., fatty acids such as retinyl laurate, retinyl myristate, retinyl palmitate, retinyl stearate, retinyl arachidate, retinyl behenate, retinyl palmitoleate, retinyl oleate, retinyl linoleate, retinyl α-linolenate, retinyl γ-linolenate, retinyl arachidonate, retinyl eicosapentaenoate, etc. In some embodiments, retinol, retinyl acetate, retinyl palmitate, retinyl propionoate, or trans-retinoic acid is complexed with a cyclodextrin.

A suitable amount of vitamin A may be used in a composition, namely about 0.1% w/w to about 4% w/w; about 0.5% w/w to about 3% w/w; about 1% w/w to about 2.5% w/w; about 1% w/w to about 2% w/w; about 2% w/w to about 3% w/w; about 0.5% w/w; about 1%; about 1.5% w/w; about 2% w/w; about 2.5% w/w; about 3% w/w; about 4% w/w; or any concentration in a range bounded by, or between, any of these values.

Cyclodextrins include natural cyclodextrins, such as α-cydodextrin, β-cyclodextrin, and γ-cyclodextrin, and their derivatives, including alkyl derivatives such as methyl-α-cyclodextrin, dimethyl-α-cyclodextrin, ethyl-α-cyclodextrin, diethyl-α-cydodextrin, propyl-α-cyclodextrin, butyl-α-cyclodextrin, pentyl-α-cyclodextrin, hexyl-α-cyclodextrin, heptyl-α-cyclodextrin, octyl-α-cyclodextrin, methyl-β-cyclodextrin, dimethyl-β-cyclodextrin, ethyl-β-cyclodextrin, diethyl-β-cyclodextrin, propyl-β-cyclodextrin, butyl-β-cyclodextrin, pentyl-β-cyclodextrin, hexyl-β-cyclodextrin, heptyl-β-cyclodextrin, octyl-β-cyclodextrin, methyl-y-cyclodextrin, dimethyl-γ-cyclodextrin, ethyl-γ-cyclodextrin, diethyl-γ-cyclodextrin, propyl-γ-cyclodextrin, butyl-γ-cyclodextrin, pentyl-γ-cyclodextrin, hexyl-γ-cyclodextrin, heptyl-γ-cyclodextrin, octyl-γ-cyclodextrin, etc.; amino group containing derivatives, including amino and aminoalkyl derivatives such as amino-α-cyclodextrin, aminomethyl-α-cyclodextrin, amino-β-cyclodextrin, aminomethyl-β-cyclodextrin, amino-γ-cyclodextrin, aminomethyl-γ-cyclodextrin, etc.; hydroxyl group containing derivatives, including hydroxyl and hydroxyalkyl derivatives such as hydroxyethyl-α-cyclodextrin, hydroxypropyl-α-cyclodextrin, dihydroxyethyl-α-cyclodextrin, dihydroxypropyl-α-cyclodextrin, etc.; carboxyl group containing derivatives, including carboxy and carboxylalkyl derivatives such as carboxymethyl-α-cyclodextrin, carboxyethyl-α-cyclodextrin, succinyl-α-cyclodextrin, carboxymethyl-β-cyclodextrin, carboxyethyl-β-cyclodextrin, succinyl-β-cyclodextrin, carboxymethyl-γ-cyclodextrin, carboxyethyl-γ-cyclodextrin, succinyl-γ-cyclodextrin, etc.; mixed ethers with hydroxylalkyl and methyl or ethyl groups, such as methyl-hydroxyethyl, ethylhydroxyethyl and ethyl-hydroxypropyl ethers of α-, β-, and γ-cyclodextrin. Other cyclodextrin derivatives may include combinations of alkyl, amino, hydroxyl, carboxyl groups and/or derivatives bearing sugar residues. Other useful cyclodextrins include maltosyl, glucosyl and maltotriosyl derivatives of β- and γ-cyclodextrin, which may contain one or more sugar residues, e.g. glucosyl or diglucosyl, maltosyl, or dimaltosyl, as well as various mixtures thereof, e.g. a mixture of maltosyl and dimaltosyl derivatives. Other useful cyclodextrin derivatives comprise anionic functional groups such as sulfobutylether derivatives, sulfonates, phosphates, and the like. In some embodiments, a cyclodextrin may be α-cydodextrin, β-cyciodextrin, γ-cyclodextrin, hydroxypropyl-α-cyclodextrin, hydroxypropyl-β-cyclodextrin, hydroxypropyl-γ-cyclodextrin, sulfobutylether-α-cyclodextrin, suifobutyiether-β-cyclodextrin, sulfobutylether-γ-cyclodextrin, hydroxyethyl-α-cyclodextrin, hydroxyethyl-β-cyclodextrin, hydroxyethyl-γ-cyclodextrin, dihydroxypropyl-α-cyclodextrin, dihydroxypropyl-β-cyclodextrin, dihydroxypropyl-γ-cyclodextrin, glucosyl-α-cyclodextrin, diglucosyl-α-cyclodextrin, maltosyl-α-cyclodextrin, glucosyl-β-cyciodextrin, diglucosyl-β-cyclodextrin, maltosyl-β-cyclodextrin, glucosyl-γ-cyclodextrin, diglucosyl-γ-cyclodextrin, maltosyl-γ-cyclodextrin, maltotriosyl-α-cyclodextrin, maltotriosyl-β-cyclodextrin, maltotriosyl-γ-cyclodextrin, dimaltosyl-α-cyclodextrin, dimaltosyl-β-cyclodextrin, dimaltosyl-γ-cyclodextrin, or mixtures thereof such as maltosyl-β-cyclodextrin/dimaltosyl-β-cyclodextrin.

Any suitable amount of cyclodextrin may be used in a composition, such as about 0.2% w/w to about 10% w/w; about 1% w/w to about 6% w/w; about 2% w/w to about 5% w/w; about 2% w/w to about 4% w/w; about 4% w/w to about 6% w/w; about 1% w/w; about 2%; about 3% w/w; about 4% w/w; about 5% w/w; about 6% w/w; about 8% w/w; or any concentration in a range bounded by, or between, any of these values.

Cyclodextrins include cyclic oligosaccharides and derivatives thereof, such as derivatives obtained by modification of a hydroxyl group of a saccharide unit such as a glucopyranoside. A cyclodextrin has a "bucket" shape, wherein the exterior side of the bucket includes hydroxyl groups from the saccharide units, or groups that are obtained by modification of these hydroxyl groups. The interior of the bucket, or the cavity, may be hydrophobic. A hydrophobic molecule, such as a hydrophobic vitamin A, may complex with a cyclodextrin through non-covalent hydrophobic interactions with the hydrophobic cavity. Thus, a vitamin A that is complexed to a cyclodextrin has at least some substantial degree of non-covalent hydrophobic interaction with a hydrophobic interior of a cavity of a cyclodextrin. For example, at equilibrium, at least about 10%, about 20%, about 50%, or about 90% of the vitamin A may have a non-covalent interaction with a hydrophobic interior of a cavity of a cyclodextrin.

A cyclodextrin and vitamin A may form a 1:1 molar complex. However, an excess of a vitamin A or a cyclodextrin may be used to shift equilibrium toward formation of complex or increase the rate of complex formation, thus maximizing complex formation. Thus, a suitable molar ratio of cyclodextrin to vitamin A may be used, depending upon the need, namely at least about 0.1:1 (cyclodextrin:vitamin A), about 0.5:1, about 1:1, or about 1.5:1; and/or up to about 5:1, about 8:1 or about 10:1. If high solubility of a vitamin A is desired, excess cyclodextrin may be used, such as a molar ratio of cyclodextrin:vitamin A that may be about 4:1 to about 1:1, about 3:1 to about 1:1, or about 2:1.

Hyaluronic acid is a non-sulfated glycosaminoglycan that enhances water retention and resists hydrostatic stresses. It is non-immunogenic and can be chemically modified in numerous fashions. Hyaluronic acid may be anionic at pH ranges around or above the pKa of its carboxylic acid groups. Unless clearly indicated otherwise, reference to hyaluronic acid, hyaluronan, or HA herein may include its fully protonated, or nonionic form as depicted below, as well as any anionic forms and salts of hyaluronic acid, such as sodium salts, potassium salts, lithium salts, magnesium salts, calcium salts, etc.

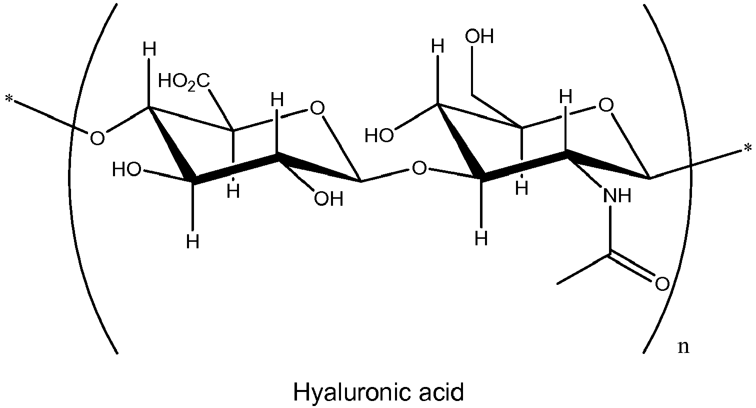

Any suitable amount of HA may be used in a composition, such as about 0.1% w/w to about 5% w/w; about 0.5% w/w to about 3% w/w; about 1% w/w to about 2.5% w/w; about 1% w/w to about 2% w/w; about 2% w/w to about 3% w/w; about 0.5% w/w; about 0.6%; about 1.2% w/w; about 1.25% w/w; about 1.3% w/w; about 1.35% w/w; about 1.4% w/w; about 1.5% w/w; about 1.75% w/w; about 2% w/w; about 2.4% w/w; about 2.5% w/w; or any concentration in a range bounded by, or between, any of these values.

An HA may have any suitable molecular weight, such as an average molecular weight of about 5,000 Da to about 20,000,000 Da; about 300,000 Da to about 800,000 Da; or about 2,000,000 Da to about 5,000,000 Da.

In some compositions, an HA may have a low molecular weight, e.g., about 100,000 Da, about 200,000 Da, about 300,000 Da, about 400,000 Da, about 500,000 Da, about 600,000 Da, about 700,000 Da, about 800,000 Da, about 900,000 Da, at most about 100,000 Da, at most about 200,000 Da, at most about 300,000 Da, at most about 400,000 Da, at most about 500,000 Da, at most about 600,000 Da, at most about 700,000 Da, at most about 800,000 Da, at most about 900,000 Da, at most about 950,000 Da, about 100,000 Da to about 500,000 Da, about 200,000 Da to about 500,000 Da, about 300,000 Da to about 500,000 Da, about 400,000 Da to about 500,000 Da, about 500,000 Da to about 950,000 Da, about 600,000 Da to about 950,000 Da, about 700,000 Da to about 950,000 Da, about 800,000 Da to about 950,000 Da, about 300,000 Da to about 600,000 Da, about 300,000 Da to about 700,000 Da, about 300,000 Da to about 800,000 Da, or about 400,000 Da to about 700,000 Da.

In some embodiments, an HA may have a high molecular weight, such as about 1,000,000 Da, about 1,500,000 Da, about 2,000,000 Da, about 2,500,000 Da, about 3,000,000 Da, about 3,500,000 Da, about 4,000,000 Da, about 4,500,000 Da, about 5,000,000 Da, at least about 1,000,000 Da, at least about 1,500,000 Da, at least about 2,000,000 Da, at least about 2,500,000 Da, at least about 3,000,000 Da, at least about 3,500,000 Da, at least about 4,000,000 Da, at least about 4,500,000 Da, at least about 5,000,000 Da, about 1,000,000 Da to about 5,000,000 Da, about 1,500,000 Da to about 5,000,000 Da, about 2,000,000 Da to about 5,000,000 Da, about 2,500,000 Da to about 5,000,000 Da, about 2,000,000 Da to about 3,000,000 Da, about 2,500,000 Da to about 3,500,000 Da, or about 2,000,000 Da to about 4,000,000 Da.

An HA may be a combination of a low molecular weight HA fraction and a high molecular weight HA fraction. A high molecular weight HA fraction can include HA having a mean molecular weight of about 1,000,000 Da or greater or 2,000,000 Da or greater, such as about 1,500,000 Da, about 2,000,000 Da, about 2,500,000 Da, about 3,000,000 Da, about 3,500,000 Da, about 4,000,000 Da, about 4,500,000 Da, or about 5,000,000 Da, or any molecular weight in a range bounded by, or between, any of these values. A low molecular weight HA fraction includes HA having a mean molecular weight of less than about 1,000,000 Da, such as about 200,000 Da, about 300,000 Da, about 400,000 Da, about 500,000 Da, about 600,000 Da, about 700,000 Da, about 800,000 Da, about 900,000 Da, or any molecular weight in a range bounded by, or between, any of these values.

In a composition comprising a low molecular weight HA fraction and a high molecular weight HA fraction, any suitable ratio of high molecular weight HA to low molecular weight HA may be used. In some embodiments the weight ratio of high molecular weight HA:low molecular weight HA may be about 20:1, about 15:1, about 10.1, about 5:1, about 1:1, about 1:15, about 1:20, about 1:5, or about 1:10.

An uncrosslinked HA fraction may also improve the rheological properties of an HA composition so as to improve treatment of a skin condition. Some compositions comprise an uncrosslinked HA where the uncrosslinked HA is present at a concentration of, e.g., about 2 mg/g, about 3 mg/g, about 4 mg/g, about 5 mg/g, about 6 mg/g, about 7 mg/g, about 8 mg/g, about 9 mg/g, about 10 mg/g, about 11 mg/g, about 12 mg/g, about 13 mg/g, about 13.5 mg/g, about 14 mg/g, about 15 mg/g, about 16 mg/g, about 17 mg/g, about 18 mg/g, about 19 mg/g, about 20 mg/g, about 40 mg/g, at least about 1 mg/g, at least about 2 mg/g, at least about 3 mg/g, at least about 4 mg/g, at least about 5 mg/g, at least about 10 mg/g, at least about 15 mg/g, at least about 20 mg/g, at least about 25 mg/g, at least about 35 mg/g, at most about 1 mg/g, at most about 2 mg/g, at most about 3 mg/g, at most about 4 mg/g, at most about 5 mg/g, at most about 10 mg/g, at most about 15 mg/g, at most about 20 mg/g, at most about 25 mg/g, about 1 mg/g to about 40 mg/g, about 7.5 mg/g to about 19.5 mg/g, about 8.8 mg/g to about 19 mg/g, about 9 mg/g to about 18 mg/g, about 10 mg/g to about 17 mg/g, about 11 mg/g to about 16 mg/g, or about 12 mg/g to about 15 mg/g. In some embodiments, the ratio of crosslinked HA to uncrosslinked HA (weight crosslinked HA:weight uncrosslinked HA) is about 1:000 to about 100:1, about 1:200 to about 2:1, or about 1:100 to about 1:20.

An HA may be crosslinked, partially crosslinked, or substantially uncrosslinked. In some embodiments, a low molecular weight HA fraction may be crosslinked, a low molecular weight HA fraction may be partially crosslinked, or a low molecular weight HA fraction may be uncrosslinked; and a high molecular weight HA fraction may be crosslinked, a high molecular weight fraction may be partially crosslinked, or a high molecular weight HA fraction may be uncrosslinked.

A crosslinked HA may result from linking two or more individual HA molecules, either directly or by a linking moiety. Crosslinking HA may increase the viscosity of an aqueous HA composition, which may result in the formation of a hydrogel in the presence of water. HA may be crosslinked using dialdehyde or disulfide crosslinking agents including, without limitation, multifunctional poly(ethylene glycol) (PEG) based crosslinking agents, divinyl sulfones, diglycidyl ethers, bis-epoxides, and biscarbodiimides. Non-limiting examples of crosslinking agents include multifunctional PEG-based crosslinking agents like pentaerythritol tetraglycidyl ether (PETGE), divinyl sulfone (DVS), 1,4-butanediol diglycidyl ether (BDDE), 1,2-bis(2,3-epoxypropoxy)ethylene (EGDGE), 1,2,7,8-diepoxyoctane (DEO), (phenylenebis-(ethyl)-carbodiimide and 1,6-hexamethylenebis(ethylcarbodiimide), adipic dihydrazide (ADH), bis(sulfosuccinimidyl)suberate (BS), hexamethylenediamine (HMDA), 1-(2,3-epoxypropyl)-2,3-epoxycyclohexane, or combinations thereof. Other useful crosslinking agents are disclosed in Stroumpoulis and Tezel, Tunably Crosslinked Polysaccharide Compositions, US2011/0077737, which is incorporated by reference in its entirety. Non-limiting methods of crosslinking HA are described in, e.g., U.S. Patent Publication 2003/0148995; U.S. Patent Publication 2008/0089918; U.S. Patent Publication 2010/0028438; U.S. Patent Publication 2006/0194758; International Patent Publication WO 2004/073759, each of which is hereby incorporated by reference in its entirety.

A hyaluronic acid and a cyclodextrin may form a conjugate wherein the hyaluronic acid and the cyclodextrin are covalently connected to one another by either one or more direct covalent bonds between the two molecules, or by a linking group covalently bonded to both the hyaluronic acid and the cyclodextrin.

Direct covalent bonding of hyaluronic acid and a cyclodextrin may be accomplished by a coupling agent, such as a bis-carbodiimide coupling agent, which may facilitate ester formation without becoming part of the linkage. Some non-limiting examples of bis-carbodiimides include N,N'-dicyclohexylcarbodiimide (DCC), N,N'-diisopropylcarbodiimide (DIC), 1-ethyl-3-(3-dimethylaminopropyl)carbodiimide (EDC), etc.

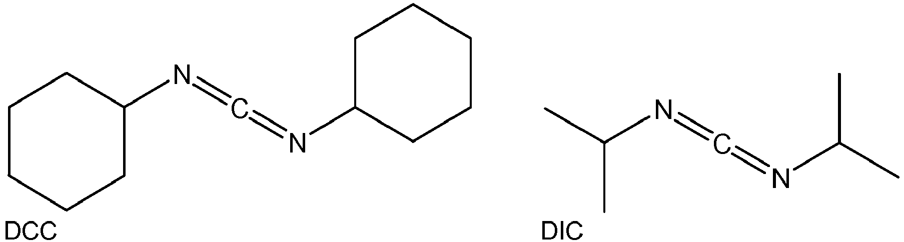

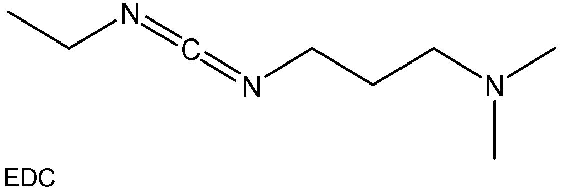

A linking group may be any moiety that can have a covalent bond to both a hyaluronic acid and a cyclodextrin. For example, a linking group may form an ester, ether, amine, amide bond, or other covalent bond to hyaluronic acid. A linking group may also form an ester, ether, amine, amide bond, or other covalent bond to a cyclodextrin. Hydroxyl groups of natural cyclodextrins (or hyaluronic acid) may be converted to a variety of functional groups to allow a broad variety of linking groups to be used. A linking group may be formed by reacting a linking agent with hyaluronic acid and a cyclodextrin. Generally, any agent that may be used to cross-link hyaluronic acid may be used as a linking agent. Some suitable linking agents may include bis-epoxides such as diglycidyl ethers, polyepoxides, diamines, polyamines, dialdehydes, disulfides crosslinking agents including, without limitation, multifunctional PEG-based crosslinking agents, divinyl sulfones, etc. Non-limiting examples of linking agents (compounds that react to form linking groups) include multifunctional PEG-based crosslinking agents like pentaerythritol tetraglycidyl ether (PETGE), divinyl sulfone (DVS), 1,4-butanediol diglycidyl ether (BDDE), 1,2-bis(2,3-epoxypropoxy)ethylene (EGDGE), 1,2,7,8-diepoxyoctane (DEO), adipic dihydrazide (ADH), bis(sulfosuccinimidyl)suberate (BS), hexamethylenediamine (HMDA), 1-(2,3-epoxypropyl)-2,3-epoxycyclohexane, etc. In some embodiments, a linking group may be butanediol diglycidyl ether, hexamethylenediamine, or pentaerythritol tetraglycidal ether.

Any suitable amount of linking agent may be used. In some embodiments, the molar ratio of active group on the linking agent to the total amount of active groups on the cyclodextrin and hyaluronic acid is about 0.5% to about 10%, about 2% to about 8%, or about 4% to about 6%. In some embodiments, about 0.5% to about 10%, about 2% to about 8%, or about 4% to about 6% of the CO₂ or OH groups on the hyaluronic acid are covalently attached to a cyclodextrin by a bond or a linking group. In some embodiments, about 0.5% to about 10%, about 2% to about 8%, or about 4% to about 6% of the OH groups on the cyclodextrin are covalently attached to a hyaluronic acid by a bond or a linking group.

Hydrogels may be formulated by using a cyclodextrin to aid in the solubilization or dispersion of a vitamin A in a hyaluronic acid-based hydrogel. A vitamin A may be complexed with a cyclodextrin and mixed with a hyaluronic acid hydrogel, or a vitamin A may be complexed with cyclodextrin in a mixture that also contains hyaluronic acid. Alternatively, cyclodextrin and hyaluronic acid may be conjugated, and a vitamin A may be complexed to the cyclodextrin portion of the conjugate. Alternatively, the cyclodextrin of an already formed cyclodextrin/vitamin A complex may be conjugated to a hyaluronic acid.

While a vitamin A/cyclodextrin complex may be formed by many different methods, in some embodiments cyclodextrin is dissolved in an organic solvent/water mixture, such as an alcohol/water mixture. Vitamin A is separately dissolved in an organic solvent/water mixture, such as an alcohol/water mixtures. The cyclodextrin solution and the vitamin A solution may then be mixed, and the solvent removed to obtain a vitamin A/cyclodextrin complex.

An HA composition may optionally comprise an anesthetic agent. An anesthetic agent may be a local anesthetic agent, including an anesthetic agent that causes a reversible local anesthesia or a loss of nociception, such as, e.g., aminoamide local anesthetics and aminoester local anesthetics. Non-limiting examples of anesthetic agents may include lidocaine, ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cyclomethycaine, dibucaine, dimethisoquin, dimethocaine, diperodon, dicyclomine, ecgonidine, ecgonine, ethyl chloride, etidocaine, β-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, combinations thereof, and salts thereof. Non-limiting examples of aminoester local anesthetics include procaine, chloroprocaine, cocaine, cyclomethycaine, dimethocaine (larocaine), propoxycaine, procaine (novocaine), proparacaine, tetracaine (amethocaine). Non-limiting examples of aminoamide local anesthetics include articaine, bupivacaine, cinchocaine (dibucaine), etidocaine, levobupivacaine, lidocaine (lignocaine), mepivacaine, piperocaine, prilocaine, ropivacaine, trimecaine, or a combination thereof.

The amount of an anesthetic agent included may be an amount effective to reduce pain experienced by an individual upon administration of the composition, such as about 0.1%, about 0.2%, about 0.3%, about 0.4%, about 0.5%, about 0.6%, about 0.7%, about 0.8% about 0.9%, about 1.0%, about 2.0%, about 3.0%, about 4.0%, about 5.0%, about 6.0%, about 7.0%, about 8.0%, about 9.0%, about 10%, at least about 0.1%, at least about 0.2%, at least about 0.3%, at least about 0.4%, at least about 0.5%, at least about 0.6%, at least about 0.7%, at least about 0.8% at least about 0.9%, at least about 1.0%, at least about 2.0%, at least about 3.0%, at least about 4.0%, at least about 5.0%, at least about 6.0%, at least about 7.0%, at least about 8.0%, at least about 9.0%, at least about 10%, at most about 0.1%, at most about 0.2%, at most about 0.3%, at most about 0.4%, at most about 0.5%, at most about 0.6%, at most about 0.7%, at most about 0.8% at most about 0.9%, at most about 1.0%, at most about 2.0%, at most about 3.0%, at most about 4.0%, at most about 5.0%, at most about 6.0%, at most about 7.0%, at most about 8.0%, at most about 9.0%, at most about 10%, about 0.1% to about 0.5%, about 0.1% to about 1.0%, about 0.1% to about 2.0%, about 0.1% to about 3.0%, about 0.1% to about 4.0%, about 0.1% to about 5.0%, about 0.2% to about 0.9%, about 0.2% to about 1.0%, about 0.2% to about 2.0%, about 0.5% to about 1.0%, or about 0.5% to about 2.0%.

Some HA compositions may comprise lidocaine, in free base or salt form (e.g. lidocaine HCI) in an amount of about 0.05% w/w to about 1% w/w; about 0.1% w/w to about 0.5% w/w, or about 0.3% w/w.

Some HA compositions do not have an anesthetic agent.

Some compositions may have a physiologically-acceptable osmolarity, e.g., about 100 mOsm/L, about 150 mOsm/L, about 200 mOsm/L, about 250 mOsm/L, about 300 mOsm/L, about 350 mOsm/L, about 400 mOsm/L, about 450 mOsm/L, about 500 mOsm/L, at least about 100 mOsm/L, at least about 150 mOsm/L, at least about 200 mOsm/L, at least about 250 mOsm/L, at most about 300 mOsm/L, at most about 350 mOsm/L, at most about 400 mOsm/L, at most about 450 mOsm/L, at most about 500 mOsm/L, about 100 mOsm/L to about 500 mOsm/L, about 200 mOsm/L to about 500 mOsm/L, about 200 mOsm/L to about 400 mOsm/L, about 300 mOsm/L to about 400 mOsm/L, about 270 mOsm/L to about 390 mOsm/L, about 225 mOsm/L to about 350 mOsm/L, about 250 mOsm/L to about 325 mOsm/L, about 275 mOsm/L to about 300 mOsm/L, or about 285 mOsm/L to about 290 mOsm/L. Osmolality agents may be used to adjust osmolality. Examples include, but are not limited to, salts such as, e.g., sodium chloride and potassium chloride; and glycerin.

Some HA compositions may be suitable for use as a dermal filler. For example, such an HA composition may be in the proper form, e.g. as a gel having an appropriate elastic modulus and viscous modulus, so as to be suitable as a dermal filler. By contrast, an HA composition that is too hard or stiff, or too liquid, may not be suitable for use as a dermal filler. Additionally, a dermal filler may have a composition that is compatible with the part of the body into which it is injected.

An HA composition may be injectable, or capable of being administered into a skin region of an individual by injection through a fine needle, such as a needle that is about 27 gauge or smaller. In some embodiments, an HA composition is injectable through a needle of, *e.g.,* about 27 gauge; about 30 gauge; about 32 gauge; about 22 gauge or smaller; about 27 gauge or smaller; about 30 gauge or smaller; about 32 gauge or smaller; about 22 gauge to about 35 gauge; about 22 gauge to about 34 gauge; about 22 gauge to about 33 gauge; about 22 gauge to about 32 gauge; about 22 gauge to about 27 gauge; or about 27 gauge to about 32 gauge.

An HA composition may optionally include, without limitation, other pharmaceutically acceptable components, including, without limitation, buffers, preservatives, tonicity adjusters, salts, antioxidants, osmolality adjusting agents, emulsifying agents, wetting agents, sweetening or flavoring agents, and the like.

Non-limiting examples of buffers include acetate buffers, borate buffers, citrate buffers, neutral buffered salines, phosphate buffers, and phosphate buffered salines. Any concentration of a buffer can be used, such as about 0.1 mM to about 900 mM. In some embodiments, an HA composition may have a pH of about 5.0 to about 8.5, about 5.0 to about 8.0, about 6.5 to about 7.5, about 7.0 to about 7.4, or about 7.1 to about 7.3.

Preservatives include, without limitation, sodium metabisulfite, sodium thiosulfate, acetylcysteine, butylated hydroxyanisole, butylated hydroxytoluene, benzalkonium chloride, chlorobutanol, thimerosal, phenylmercuric acetate, phenylmercuric nitrate, a stabilized oxy chloro composition, such as, *e.g*., PURITE® (Allergan, Inc. Irvine, CA) and chelants, such as, *e.g*., DTPA or DTPA-bisamide, calcium DTPA, and CaNaDTPA-bisamide.

An HA composition may be used to treat a soft tissue condition of an individual. As used herein, the term "treating," includes reducing any detectable amount, or eliminating in an individual a cosmetic or clinical symptom of a soft tissue condition characterized by a soft tissue imperfection, defect, disease, and/or disorder; or delaying or preventing in an individual the onset of a cosmetic or clinical symptom of a condition characterized by a soft tissue imperfection, defect, disease, and/or disorder. For example, the term treating includes reducing a symptom of a condition characterized by a soft tissue defect, disease, and/or disorder by any detectable amount. In some embodiments, a symptom may be reduced at least about 20%, at least about 30%, at least about 40%, at least about 50%, at least about 60%, at least about 70%, at least about 80%, at least about 90% or at least about 100%. The effectiveness of a hydrogel composition disclosed herein in treating a condition characterized by a soft tissue defect, disease, and/or disorder can be determined by observing one or more cosmetic, clinical symptoms, and/or physiological indicators associated with the condition. An improvement in a soft tissue defect, disease, and/or disorder also can be indicated by a reduced need for a concurrent therapy. Those of skill in the art will know the appropriate symptoms or indicators associated with a specific soft tissue defect, disease, and/or disorder and will know how to determine if an individual is a candidate for treatment with a compound or composition disclosed herein.

A soft tissue condition may include, without limitation, a soft tissue imperfection, defect, disease, and/or disorder, such as a breast imperfection, defect, disease and/or disorder, such as, *e.g.,* a breast augmentation, a breast reconstruction, mastopexy, micromastia, thoracic hypoplasia, Poland's syndrome, defects due to implant complications like capsular contraction and/or rupture; a facial imperfection, defect, disease or disorder, such as, *e.g.,* a facial augmentation, a facial reconstruction, a mesotherapy, Parry-Romberg syndrome, lupus erythematosus profundus, dermal divots, scars, sunken cheeks, thin lips, nasal imperfections or defects, retro-orbital imperfections or defects, a facial fold, line and/or wrinkle like a glabellar line, a nasolabial line, a perioral line, and/or a marionette line, and/or other contour deformities or imperfections of the face; a neck imperfection, defect, disease or disorder; a skin imperfection, defect, disease and/or disorder; other soft tissue imperfections, defects, diseases and/or disorders, such as, *e.g.,* an augmentation or a reconstruction of the upper arm, lower arm, hand, shoulder, back, torso including abdomen, buttocks, upper leg, lower leg including calves, foot including plantar fat pad, eye, genitals, or other body part, region or area, or a disease or disorder affecting these body parts, regions or areas. As used herein, the term "mesotherapy" includes a non-surgical cosmetic treatment technique of the skin involving intra-epidermal, intra-dermal, and/or subcutaneous injection of an agent administered as small multiple droplets into the epidermis, dermoepidermal junction, and/or the dermis.

The amount of an HA composition used may be determined based on the alteration and/or improvement desired, the reduction and/or elimination of a soft tissue condition symptom desired, the clinical and/or cosmetic effect desired by the individual and/or physician, and the body part or region being treated. The effectiveness of composition administration may be manifested by one or more of the following clinical and/or cosmetic measures: altered and/or improved soft tissue shape, altered and/or improved soft tissue size, altered and/or improved soft tissue contour, altered and/or improved tissue function, tissue ingrowth support and/or new collagen deposition, sustained engraftment of composition, improved patient satisfaction and/or quality of life, and decreased use of implantable foreign material.

For example, effectiveness of the compositions and methods in treating a facial soft tissue may be manifested by one or more of the following clinical and/or cosmetic measures: increased size, shape, and/or contour of facial feature like increased size, shape, and/or contour of lip, cheek or eye region; altered size, shape, and/or contour of facial feature like altered size, shape, and/or contour of lip, cheek or eye region shape; reduction or elimination of a wrinkle, fold or line in the skin; resistance to a wrinkle, fold or line in the skin; rehydration of the skin; increased elasticity to the skin; reduction or elimination of skin roughness; increased and/or improved skin tautness; reduction or elimination of stretch lines or marks; increased and/or improved skin tone, shine, brightness and/or radiance; increased and/or improved skin color, reduction or elimination of skin paleness; sustained engraftment of composition; decreased side effects; improved patient satisfaction and/or quality of life.

In some embodiments, the amount of a hydrogel composition administered is, *e.g.,* about 0.01 g, about 0.05 g, about 0.1 g, about 0.5 g, about 1 g, about 5 g, about 10 g, about 20 g, about 30 g, about 40 g, about 50 g, about 60 g, about 70 g, about 80 g, about 90 g, about 100 g, about 150 g, or about 200 g, about 0.01 g to about 0.1 g, about 0.1 g to about 1 g, about 1 g to about 10 g, about 10 g to about 100 g, about 50 g to about 200 g, about 0.01 mL, about 0.05 mL, about 0.1 mL, about 0.5 mL, about 1 mL, about 5 mL, about 10 mL, about 20 mL, about 30 mL, about 40 mL, about 50 mL, about 60 mL, about 70 g, about 80 mL, about 90 mL, about 100 mL, about 150 mL, or about 200 mL, about 0.01 mL to about 0.1 mL, about 0.1 mL to about 1 mL, about 1 mL to about 10 mL, about 10 mL to about 100 mL, or about 50 mL to about 200 mL.

Duration of treatment may be determined based on the cosmetic and/or clinical effect desired by the individual and/or physician and the body part or region being treated. For some treatments, administration of an HA composition can effectively treat a soft tissue condition for, *e.g.,* about 6 months, about 7 months, about 8 months, about 9 months, about 10 months, about 11 months, about 12 months, about 13 months, about 14 months, about 15 months, about 18 months, or about 24 months, at least about 6 months, at least about 7 months, at least about 8 months, at least about 9 months, at least about 10 months, at least about 11 months, at least about 12 months, at least about 13 months, at least about 14 months, at least about 15 months, at least about 18 months, or at least about 24 months, about 6 months to about 12 months, about 6 months to about 15 months, about 6 months to about 18 months, about 6 months to about 21 months, about 6 months to about 24 months, about 9 months to about 12 months, about 9 months to about 15 months, about 9 months to about 18 months, about 9 months to about 21 months, about 6 months to about 24 months, about 12 months to about 15 months, about 12 months to about 18 months, about 12 months to about 21 months, about 12 months to about 24 months, about 15 months to about 18 months, about 15 months to about 21 months, about 15 months to about 24 months, about 18 months to about 21 months, about 18 months to about 24 months, or about 21 months to about 24 months.

An HA composition may be administered to a skin region of an individual by injection into a dermal region or a hypodermal region, such as an epidermal-dermal junction region, a papillary region, a reticular region, or any combination thereof.

As used herein, the term "dermal region" refers to the region of skin comprising the epidermal-dermal junction and the dermis including the superficial dermis (papillary region) and the deep dermis (reticular region). The skin is composed of three primary layers: the epidermis, which provides waterproofing and serves as a barrier to infection; the dermis, which serves as a location for the appendages of skin; and the hypodermis (subcutaneous adipose layer). The epidermis contains no blood vessels, and is nourished by diffusion from the dermis. The main type of cells which make up the epidermis are keratinocytes, melanocytes, Langerhans cells and Merkels cells.

The dermis includes the layer of skin beneath the epidermis that includes connective tissue and cushions the body from stress and strain. The dermis is tightly connected to the epidermis by a basement membrane. It also harbors many mechanoreceptor/nerve endings that provide the sense of touch and heat. It contains the hair follicles, sweat glands, sebaceous glands, apocrine glands, lymphatic vessels and blood vessels. The blood vessels in the dermis provide nourishment and waste removal from its own cells as well as from the Stratum basale of the epidermis. The dermis is structurally divided into two areas: a superficial area adjacent to the epidermis, called the papillary region, and a deep thicker area known as the reticular region.

The papillary region is composed of loose areolar connective tissue. It is named for its fingerlike projections called papillae that extend toward the epidermis. The papillae provide the dermis with a "bumpy" surface that interdigitates with the epidermis, strengthening the connection between the two layers of skin. The reticular region lies deep in the papillary region and is usually much thicker. It is composed of dense irregular connective tissue, and receives its name from the dense concentration of collagenous, elastic, and reticular fibers that weave throughout it. These protein fibers give the dermis its properties of strength, extensibility, and elasticity. Also located within the reticular region are the roots of the hair, sebaceous glands, sweat glands, receptors, nails, and blood vessels. Tattoo ink is held in the dermis. Stretch marks from pregnancy are also located in the dermis.

The hypodermis lies below the dermis. Its purpose is to attach the dermal region of the skin to underlying bone and muscle as well as supplying it with blood vessels and nerves. It includes loose connective tissue and elastin. The main cell types are fibroblasts, macrophages and adipocytes (the hypodermis contains 50% of body fat).

Some methods of treating a soft tissue condition of an individual comprise administering an HA composition to a site of a soft tissue condition of the individual to improve or treat the soft tissue condition. In some embodiments a soft tissue condition includes a breast tissue condition, a facial tissue condition, a neck condition, a skin condition, an upper arm condition, a lower arm condition, a hand condition, a shoulder condition, a back condition, a torso including abdominal condition, a buttock condition, an upper leg condition, a lower leg condition including calf condition, a foot condition including plantar fat pad condition, an eye condition, a genital condition, or a condition effecting another body part, region or area.

Some methods of treating a skin condition comprise administering an HA composition to an individual suffering from a skin condition to improve or treat the skin condition. Skin dehydration may be treated by administering an HA composition to an individual suffering from skin dehydration to rehydrate the skin, thereby treating skin dehydration. A lack of skin elasticity may be treated by administering an HA composition to an individual suffering from a lack of skin elasticity to increase the elasticity of the skin, thereby treating a lack of skin elasticity. Skin roughness may be treated by administering an HA composition to decrease skin roughness, thereby treating skin roughness. A lack of skin tautness may be treated by administering an HA composition to an individual suffering from a lack of skin tautness to make the skin tauter, thereby treating a lack of skin tautness.

A skin stretch line or mark may be treated by administering an HA composition to an individual suffering from a skin stretch line or mark to reduce or eliminate the skin stretch line or mark, thereby treating a skin stretch line or mark. Skin paleness may be treated by administering an HA composition to an individual suffering from skin paleness to increase skin tone or radiance, thereby treating skin paleness. Skin wrinkles may be treated by administering an HA composition to an individual suffering from skin wrinkles to reduce or eliminate skin wrinkles or makes the skin resistant to skin wrinkles, thereby treating skin wrinkles.

Some methods of treating a skin condition comprise administering an HA composition into a dermal region of the individual, to improve the skin condition. Skin conditions treated by the disclosed compositions include, without limitation, augmentations, reconstructions, diseases, disorders, defects, or imperfections of a body part, region or area. In one aspect, a skin condition treated by the disclosed compositions include, without limitation, a facial augmentation, a facial reconstruction, a facial disease, a facial disorder, a facial defect, or a facial imperfection. In one aspect, a skin condition treated by the disclosed compositions can include, without limitation, skin dehydration, a lack of skin elasticity, skin roughness, a lack of skin tautness, a skin stretch line or mark, skin paleness, a dermal divot, a sunken cheek, a thin lip, a retro-orbital defect, a facial fold, or a wrinkle.

### EXAMPLE 1

Low molecular weight hyaluronic acid or high molecular weight hyaluronic acid (400 mg) and cyclodextrin (800 mg) are hydrated in 1800 mg of 1 wt% NaOH in a syringe for about 30 min. In a separate syringe, 120 mg of BDDE and 600 mg of 1 wt% NaOH are added. The two syringes are connected via a syringe connector. The components in above two syringes are mixed about 20 times by passing the contents back and forth between the 2 syringes. The mixed paste is put in a vial and in a 50°C water bath for about 2.5 hours. After the gel is formed, 9.05 gm of HCI-PBS (58.4 mg of 12N HCI in 9.0 gm of PBS buffer) is added to neutralize and swell the gel. The gel is sized through a ∼60 µm screen and mixed about 20 times by passing the contents back and forth between 2 syringes. The gel is put in a 15,000 MWCO RC dialysis bag and dialyzed in PBS, a pH 7.4 buffer. The dialysis is continued for 3 days, with frequent change of PBS buffer. After the dialysis, the gel is put in a syringe and stored in a 4 °C refrigerator.

### EXAMPLE 2

The procedure is similar to Example 1, except that pentaerythritol glycidal ether (30 mg) is used as a crosslinker instead of BDDE.

### EXAMPLE 3

Retinol (20 mg) is dissolved in isopropanol (5 ml). Then mixed with HA-cyclodextrin hydrogel (2.0 gm) of Example 1. The hydrogel is dialyzed against PBS buffer and stored in a 4°C refrigerator.

### EXAMPLE 4

Retinyl acetate is (20 mg) dissolved in isopropanol (8 ml). Then mixed with HA-cyclodextrin hydrogel (2.0 gm) of Example 1. The hydrogel is dialyzed against PBS buffer and stored in a 4°C refrigerator.

### EXAMPLE 5

Retinol (20 mg) is dissolved in isopropanol (5 ml). Then mixed with HA-cyclodextrin hydrogel (3.0 gm) of Example 2. The hydrogel is dialyzed against PBS buffer and stored in a 4°C refrigerator.

### EXAMPLE 6

Retinyl acetate (20 mg) is dissolved in isopropanol (5 ml). Then mixed with HA-cyclodextrin hydrogel (5.0 gm) of Example 2. The hydrogel is dialyzed against PBS buffer and stored in a 4°C refrigerator.

### EXAMPLE 7

Solution A is prepared as follows. β-cydodextrin (113.5 mg) is dissolved in 50 mL of water to yield a 2 mM solution of the β-cydodextrin. Ethanol was added drop wise to this solution to yield Solution A.

Solution B is prepared as follows. Retinyl palmitate (52.49 mg) is dissolved in 100 ml of ethanol to yield a 1 mM solution of retinal palmitate. Water is added to form the Solution B.

Solution A and Solution B are mixed and the ethanol is removed to obtain a retinyl palmitate/β cyclodextrin complex dissolved in water.

### EXAMPLE 8

Low molecular weight hyaluronic acid or high molecular weight hyaluronic acid (400 mg) and β-cyclodextrin/retinyl acetate complex (1:1) (800 mg) are hydrated in 1800 mg of 1 wt% NaOH in a syringe for about 30 min. In a separate syringe, 120 mg of BDDE and 600 mg of 1 wt% NaOH are added. The two syringes are connected via a syringe connector. The components in above two syringes are mixed about 20 times by passing the contents back and forth between the 2 syringes. The mixed paste is put in a vial and in a 50°C water bath for about 2.5 hours. After the gel is formed, 9.05 gm of HCI-PBS (58.4 mg of 12N HCI in 9.0 gm of PBS buffer) is added to neutralize and swell the gel. The gel is sized through a ∼60 µm screen and mixed about 20 times by passing the contents back and forth between 2 syringes. The gel is put in a 15,000 MWCO RC dialysis bag and dialyzed in PBS, a pH 7.4 buffer. The dialysis is continued for 3 days, with frequent change of PBS buffer. After the dialysis, the gel is put in a syringe and stored in a 4 °C refrigerator.

### EXAMPLE 9

### Treatment of Facial Defects of the Cheek

This example illustrates the use of compositions and methods disclosed herein for a facial disorder.

A 58-year-old woman presents with a lean face. She feels her face looks old, sad and bitter because of the less fullness of her cheek contour. Pre-operative evaluation of the person includes routine history and physical examination in addition to thorough informed consent disclosing all relevant risks and benefits of the procedure. The physician evaluating the individual determines that she is a candidate for administration of the dermal filler compositions and methods disclosed herein.

A composition of the invention, such as described in EXAMPLE 8, is provided in a 20 mL syringe. One-holed blunt infiltration cannulas (3 mm inner diameter) are used to place about 15 mL of the composition in the syringe subcutaneously and under superficial musculoaponeurotix system into the left and right checks.

The individual is monitored for approximately 7 days. The physician evaluates the treatment area and determines that the treatment was successful. The woman's cheeks are fuller than prior to treatment. Both the woman and her physician are satisfied with the results of the procedure because she looks younger than she did when she came in for treatment.

### EXAMPLE 10

### Treatment of Facial Defects of Eyelids

This example illustrates the use of compositions and methods disclosed herein for a treatment of eyelid defects.

A 37-year-old woman presents with fine wrinkles around her eyes and she reports that her eyes make her look old and angry. Pre-operative evaluation of the person includes routine history and physical examination in addition to thorough informed consent disclosing all relevant risks and benefits of the procedure. The physician evaluating the individual determines that she is a candidate for administration of the dermal filler compositions and methods disclosed herein.

A composition, such as made as described in Example 8, is provided in a 20 mL syringe. About 2.5 mL of the composition is injected with a fine needle subcutaneously in the skin beneath the wrinkles into the regions adjacent the eyes.

The individual is monitored for approximately 7 days. The physician evaluates the eye of the patient and determines that the treatment was successful. Both the woman and her physician are satisfied with the results of the procedure because her eyes appear refreshed and the skin appears rejuvenated. Approximately one year after the procedure, the woman indicates that her quality of life has improved.

### EXAMPLE 11

### Treatment of acne scars

This example illustrates the use of compositions and methods disclosed herein for treatment of acne scars.

A 25-year-old man presents with moderate acne scarring on his jaw line including depressions and pitting. He reports that he is dissatisfied with his appearance and feels he is socially inhibited due to his perception of his appearance. Pre-operative evaluation of the person includes routine history and physical examination in addition to thorough informed consent disclosing all relevant risks and benefits of the procedure. The physician evaluating the individual determines that he is a candidate for administration of the dermal filler compositions and methods disclosed herein.

A composition such as that made as described in Example 8, is provided in 10 mL syringes. The physician injects a small amount of the composition below the skin in each depressed or pitted area of the patient's jawline to raise the area to match the surrounding skin.

The individual returns for a follow up visit with the physician in 14 days. The physician evaluates the patient and determines that the treatment was successful. The man reports he is satisfied with the results of the procedure because his skin is more smooth in appearance and the acne scarring is substantially less visible. Approximately six months after the procedure, the man returns for a follow up treatment. He reports to the physician that his quality of life has greatly improved since the procedure and he is no longer shy about his appearance.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

The terms "a," "an," "the" and similar referents used in the context of describing the invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of any claim. No language in the specification should be construed as indicating any non-claimed element essential to the practice of the invention.

Groupings of alternative elements or embodiments disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

## Claims

1. A dermal aesthetic hydrogel product comprising:
(a) a hyaluronic acid;
(b) a cyclodextrin; and
(c) a vitamin A;
wherein the vitamin A is complexed to the cyclodextrin, the amount of vitamin A is between 0.1% and 4% by weight of the product, and the cyclodextrin and the vitamin A have a molar ratio to each other of 0.1:1 to 10:1.

2. The product of claim 1, wherein the cyclodextrin and the vitamin A have a molar ratio to each other of 1:1 to 2:1.

## Patentansprüche

1. Dermales ästhetisches Hydrogelprodukt, umfassend:
(a) eine Hyaluronsäure;
(b) ein Cyclodextrin; und
(c) ein Vitamin A;
wobei das Vitamin A mit dem Cyclodextrin komplexiert ist, der Gehalt an Vitamin A zwischen 0,1 % und 4 % nach Gewicht des Produkts beträgt, und das Cyclodextrin und das Vitamin A ein Molverhältnis zueinander von 0,1:1 bis 10:1 aufweisen.

2. Produkt gemäß Anspruch 1, wobei das Cyclodextrin und das Vitamin A ein Molverhältnis zueinander von 1:1 bis 2:1 aufweisen.

## Revendications

1. Produit d'hydrogel dermique esthétique comprenant :
(a) un acide hyaluronique ;
(b) une cyclodextrine ; et
(c) une vitamine A ;
dans lequel la vitamine A est complexée avec la cyclodextrine, la quantité de vitamine A se situe entre 0,1% et 4 % en poids du produit et la cyclodextrine et la vitamine A ont un rapport molaire mutuel de 0,1:1 à 10:1.

2. Produit selon la revendication 1, dans lequel la cyclodextrine et la vitamine A ont un rapport molaire mutuel de 1:1 à 2:1.
